# EUROPEAN PATENT APPLICATION

(11) **EP 4 768 014 A1**
(43) Date of publication of application: **01.07.2026**
(21) Application number: 24307295.6
(22) Date of filing: 24.12.2024
(51) Int. Cl.: A61K 8/60, A61K 8/34, A61P 17/02, A61Q 11/00, A61Q 17/00

(54) **NON-THERAPEUTIC USE OF ISOSORBIDE AS A SOOTHING AGENT**

(71) Applicant: ROQUETTE FRERES, 62136 Lestrem (FR)
(72) Inventor: MENTINK, Léon, 59000 LILLE (FR); WILS, Daniel, 59190 MORBECQUE (FR)
(74) Representative: Roquette IP International

(57) **Abstract**

The present application is about the non-therapeutic use of 1,4:3,6-dianhydrohexitols, preferably isosorbide, as a soothing agent in cosmetic compositions. Isosorbide is able to reduce or eliminate at least one non-pathological physical sensation chosen from burning sensations, warmth, tightness, tingling, numbness and/or itching, said sensation occurring on the skin and/or on the oral mucosa. Isosorbide may be applied as a cosmetic care to alleviate these non-pathological sensations either before, during or after their occurrence, as a dedicated care or inside a cosmetic product containing factors of chemical aggression, such as surfactant or organic acids.

## Description

### TECHNICAL FIELD

The present disclosure relates to non-therapeutic use of a 1 ,4:3,6-dianhydrohexitol, notably isosorbide, as a soothing agent.

### BACKGROUND ART

In the field of personal care and cosmetics, there is a constant need to develop products capable of relieving minor aggressions and improving users' overall comfort. These aggressions can affect various parts of the body, particularly the skin and the oral sphere, notably the mucous membranes, and can be caused by a multitude of factors.

Skin serves as the body's primary protective barrier against the environment, making it particularly responsive to various external influences. In our modern daily lives, the skin, even healthy, regularly encounters multiple sources of aggression that can trigger temporary, non-pathological physical sensations. The most common of these are chemical aggressions, which occur during routine personal care and household activities. Whether using cosmetic products containing surfactants like Sodium Lauryl Sulfate, applying active skincare ingredients such as α-hydroxy acids, β-hydroxy acids, or retinoids, or simply carrying out household chores with cleaning agents and detergents, healthy skin naturally responds to these chemical contacts. Similarly, our increasingly urban lifestyle exposes skin to various environmental aggressions throughout the day. From morning commutes where skin faces temperature fluctuations and wind exposure, to outdoor activities, through to exposure to urban pollution including ozone and cigarette smoke, skin continuously adapts to changing environmental conditions. Physical activities and daily grooming routines also subject skin to mechanical aggressions: the gentle friction of clothing, the regular actions of cleansing and exfoliation, the process of shaving or hair removal, and even the simple act of towel drying or applying skincare products. Beyond these external factors, the skin also responds to internal influences. Natural processes such as hormonal variations, aging, and emotional states, including stress and anxiety, can trigger temporary sensations in healthy skin.

The oral mucosa, while less exposed to the external environment than skin, faces its own unique set of daily challenges. Throughout the day, the delicate tissues of the oral cavity encounter various influences that can trigger temporary, non-pathological physical sensations. During oral hygiene routines, the mucosa comes into contact with chemical agents present in toothpastes and mouthwashes, including surfactants and active ingredients. The mechanical actions of daily oral care, from tooth brushing and flossing to the wearing of dental appliances, create regular physical interactions with these sensitive tissues. The oral mucosa also experiences frequent temperature variations and chemical stimulation through our eating and drinking habits, whether enjoying a hot cup of coffee, consuming spicy foods, or sipping cold beverages. Environmental factors play their part too, particularly in air-conditioned environments or at different altitudes, where changes in air pressure and humidity can affect oral comfort. Similar to skin, the oral mucosa also responds to endogenous factors, with hormonal fluctuations, age-related changes, and stress-induced responses all contributing to temporary sensations in these healthy tissues.

In response to these various daily challenges, both skin and oral mucosa exhibit a range of temporary, non-pathological physical sensations. These natural responses may manifest as a transient feeling of warmth or a mild burning sensation, much like the temporary warmth felt after applying a facial cream. Sometimes, the tissues might experience a gentle tingling or a slight numbness, similar to the temporary sensation felt after using a refreshing mouthwash. Occasional tightness might occur, comparable to the sensation experienced after washing one's face, while brief episodes of itching may arise as a natural response to environmental changes. In the oral cavity specifically, these sensations might temporarily affect comfort during daily activities like eating or speaking, but they remain within the realm of normal tissue responses. Importantly, all these sensations are temporary and represent the natural adaptability of healthy tissues to their dynamic environment.

Nonetheless, faced with these issues, there is a real need to develop compounds capable of calming the effect(s) of these aggressions and soothing these unpleasant sensations, both in the skin and the oral sphere. It is crucial to emphasize that the objective is not to create a therapeutic treatment, but rather a cosmetic solution intended for healthy individuals without any particular physical problem. The challenge is to find a compound that can quickly relieve sensations of discomfort, be incorporated into various cosmetic formulations without altering their properties and be well tolerated by all types of skin and mucous membranes, including the most sensitive. Furthermore, for the oral sphere, this compound should ideally not disturb the balance of the oral flora, have a pleasant or neutral taste, and not interfere with normal mouth functions such as salivation or flavor perception.

In conclusion, the development of such a soothing compound would represent a significant advancement in the field of cosmetic care, offering healthy consumers an effective solution to improve their daily comfort and skin and oral well-being. This innovation would respond to a growing need for products capable of protecting and soothing sensitive tissues against the numerous aggressions of modern life, while respecting the physiological integrity of the skin and oral sphere.

### SUMMARY

The inventors have now surprisingly discovered that 1,4:3,6-dianhydrohexitols, preferably isosorbide, exhibit remarkable soothing properties when applied to both skin and oral mucosa. It is important to emphasize that 1,4:3,6-dianhydrohexitols are specifically intended for cosmetic use in healthy individuals without any pathological conditions and is not meant for therapeutic purposes nor for preventing any disease or pathological condition. 1 ,4:3,6-dianhydrohexitols, preferably isosorbide, provide a pleasant soothing effect that helps maintain comfort in everyday situations where skin or oral mucosa may experience temporary discomfort from common environmental or lifestyle factors. 1,4:3,6-dianhydrohexitols, preferably isosorbide, demonstrate excellent tolerability in healthy subjects and can be readily incorporated into various cosmetic formulations without compromising their effectiveness or the stability of the formulation.

This discovery represents a significant advancement in purely cosmetic applications, offering a solution exclusively for healthy individuals seeking to enhance their daily comfort, without any therapeutic or preventive medical purpose.

Furthermore, when used in oral applications in healthy individuals, these compounds respect the natural oral flora and are compatible with normal oral functions, all while remaining strictly within the scope of cosmetic applications. This clear distinction from both therapeutic and preventive medical applications, combined with their effectiveness in providing comfort to healthy individuals, makes these compounds particularly valuable for the cosmetic industry.

Hence, the present invention relates to the non-therapeutic use of a 1,4:3,6-dianhydrohexitol, preferably isosorbide, as a soothing agent.

### DETAILED DESCRIPTION

The present invention relates to the non-therapeutic use of a 1,4:3,6-dianhydrohexitol, preferably isosorbide, as a soothing agent.

### 1,4:3,6-dianhydrohexitols

1,4:3,6-dianhydrohexitols, also known simply as dianhydrohexitols or isohexides, are products of double internal dehydration of C6 polyols (hexitols) such as sorbitol, mannitol, and iditol. In this application, the term "1,4:3,6-dianhydrohexitol" encompasses isosorbide (1,4:3,6-dianhydrosorbitol), isomannide (1,4:3,6-dianhydromannitol), and isoidide (1,4:3,6-dianhydroiditol).

According to one embodiment, the 1,4:3,6-dianhydrohexitol is isosorbide.

According to another embodiment, the 1,4:3,6-dianhydrohexitol is a mixture of at least two 1,4:3,6-dianhydrohexitols chosen from isosorbide, isomannide, and isoidide.

### Soothing agent

A soothing agent as defined in the context of this invention, refers to a compound, the 1,4:3,6-dianhydrohexitol, preferably the isosorbide, intended for cosmetic use in healthy individuals without any pathological conditions. It is important to emphasize that a soothing agent is not meant for therapeutic purposes nor for preventing any disease or pathological condition. Instead, it provides a pleasant soothing effect that helps maintain comfort in everyday situations where the skin or oral mucosa may experience temporary discomfort from common environmental or lifestyle factors or endogenous factors.

Hence, and as used herein a "soothing agent" is defined as a compound that provides immediate comfort and relief from temporary non-pathological physical sensation in healthy skin and oral mucosa, exclusively for cosmetic purposes. Such an agent helps maintain the well-being of healthy individuals experiencing temporary non-pathological physical sensation due to everyday environmental or lifestyle factors, without any therapeutic or preventive medical purpose.

In an embodiment, the soothing agent is a soothing agent for the skin and/or the oral mucosa.

In a preferred embodiment, the soothing agent is a soothing agent for a healthy skin and/or a healthy oral mucosa.

In an embodiment, the soothing agent is a soothing agent for the skin, preferably a healthy skin.

"Healthy skin" refers to skin that is free from any pathological conditions or diseases. It is characterized by its normal function and appearance, without any signs of irritation, inflammation, or other abnormalities. Healthy skin maintains its integrity and resilience against everyday environmental and lifestyle factors but can however suffer from at least one non-pathological physical sensation chosen from burning sensations, warmth, tightness, tingling, numbness, and/or itching.

In an embodiment, the soothing agent is a soothing agent for the oral mucosa, preferably a healthy oral mucosa.

The oral mucosa refers to the mucous membranes lining the inside of the mouth. It includes the inner surfaces of the cheeks, lips, tongue, floor of the mouth, and the roof of the mouth (palate).

"Healthy oral mucosa" refers to the mucous membranes lining the inside of the mouth that are free from any pathological conditions or diseases. It is characterized by its normal function and appearance, without any signs of irritation, inflammation, or other abnormalities. Healthy oral mucosa maintains its integrity and resilience against everyday environmental and lifestyle factors. However, healthy oral mucosa can suffer from at least one non-pathological physical sensation chosen from burning sensations, warmth, tightness, tingling, numbness, and/or itching.

### Non-pathological physical sensation

In an embodiment, the soothing agent reduces or eliminates at least one non-pathological physical sensation chosen from burning sensations, warmth, tightness, tingling, numbness, and/or itching.

Non-pathological physical sensations refer to temporary discomforts experienced by healthy skin or oral mucosa that are not associated with any underlying disease or pathological condition. These sensations can arise from everyday environmental or lifestyle factors.

A burning sensation is usually a feeling of heat on healthy skin or mucosa, often described as a mild to moderate stinging or scalding sensation.

The sensation of warmth is an increased temperature sensation in a localized area of healthy skin or mucosa, which may or may not be accompanied by redness, and preferably which is not accompanied by redness.

The sensation of tightness is a feeling of constriction or lack of elasticity in healthy skin or mucosa, often leading to discomfort or a sensation of being stretched.

The sensation of tingling is a prickling or slight stinging sensation on healthy skin or mucosa, similar to the feeling of "pins and needles."

The sensation of numbness is a loss of sensation or a reduced ability to feel in a specific area of healthy skin or mucosa.

The sensation of itching is a temporary sensation that provokes the desire to scratch the affected area of healthy skin or mucosa.

### Endogenous and exogenous stress

In an embodiment, the non-pathological physical sensation is induced by endogenous and/or exogenous stress.

### Exogenous stress

Exogenous stress refers to external factors that can cause temporary non-pathological physical sensations, as described hereinbefore, to healthy skin or oral mucosa without leading to any pathological conditions.

These factors can include chemical aggressions, mechanical aggressions, environmental aggressions, aggressions triggered by foods and/or beverages and social relationships and/or psychological issues:

Chemical aggressions are the aggressions caused by exposure to substances such as:

For healthy skin:
- Cosmetic ingredients selected among fragrances, preservatives, colorants, active principles such as alpha-hydroxy acids (AHAs) or retinoids, surfactants, generally found in cleansing products like shampoos and body washes and notably Sodium Lauryl Sulfate (SLS)).
- Household chemicals (cleaning agents, detergents, solvents).

For healthy oral mucosa:
- Oral care product ingredients selected among surfactants and notably Sodium Lauryl Sulfate (SLS), preservatives such as sodium benzoate, potassium sorbate, flavoring agents such as menthol, thymol, eucalyptol, active ingredients such as calcium carbonate, zinc citrate, potassium nitrate, fluoride salts, pyrophosphate.

Typically, chemical aggressions can lead to non-pathological physical sensations:
- On healthy skin: primarily burning sensations and tightness, with occasional tingling. Contact with cosmetic ingredients or household products can temporarily disrupt skin comfort, leading to mild burning sensations, while tightness often follows as a natural response of healthy skin.
- On healthy oral mucosa: primarily tingling and burning sensations. Contact with oral care products containing surfactants typically triggers temporary tingling, followed by mild burning sensations as a normal response of healthy oral tissue.

Mechanical agressions include:
- On healthy skin: friction from clothing or accessories, shaving, exfoliation, brushing, hair removal (waxing, epilating), towel drying, massage, face scrubbing, application and removal of cosmetic products, mechanical peeling.
- On healthy oral mucosa: dental flossing, teeth brushing, wearing dental appliances, chewing, use of oral hygiene tools.

Typically, mechanical aggressions can lead to non-pathological physical sensations:
- On healthy skin: primarily tingling and tightness, with occasional burning sensations. Physical actions create temporary tingling, often followed by mild tightness.
- On healthy oral mucosa: primarily tingling and numbness. Mechanical stimulation typically causes temporary tingling sensations, sometimes accompanied by mild numbness.

Environmental aggressions are external conditions affecting healthy skin and oral mucosa, including:
- For healthy skin: exposure to temperature variations (cold air/wind, hot air), extreme weather conditions (humidity variations, atmospheric pressure changes), air pollution (particulate matter, smog, and saltwater exposure
- For healthy oral mucosa: exposure to temperature variations (breathing cold or hot air), changes in air humidity (dry air, mouth breathing), and atmospheric conditions (altitude changes, air conditioning).

Typically, environmental aggressions can lead to non-pathological physical sensations:
- On healthy skin: sensations chosen among tightness, warmth, itching, numbness, and burning sensations. Exposure to factors like wind or low humidity can result in temporary sensation of tightness and warmth, while pollution exposure may cause mild itching. Temperature variations may induce temporary numbness in response to cold, or burning sensations accompanied by warmth in response to heat.
- On healthy oral mucosa: sensations chosen among numbness, tingling, and dryness. Exposure to dry air or temperature variations can lead to temporary sensations of dryness or numbness, while rapid temperature changes may cause tingling sensations.

The aggressions triggered by foods and beverages are those primarily affecting healthy oral mucosa, and are those caused by:
- Spicy foods and beverages,
- Acidic foods and beverages,
- Temperature extremes (very hot or cold items).

Typically, food and beverage aggressions lead to non-pathological physical sensations primarily in healthy oral mucosa: burning sensations from spicy foods, tingling from acidic items, and numbness from very cold items.

Social relationships and psychological issues affecting healthy skin are:
- Stress-induced perspiration,
- Tension-related muscle tightness affecting skin,
- Emotional flushing.

Social relationships and psychological issues affecting healthy oral mucosa are:
- Stress-related dry mouth,
- Tension in jaw and oral muscles.

Typically, psychological factors can lead to non-pathological physical sensations:
- On healthy skin: primarily warmth and tightness, with occasional itching during stress responses,
- On healthy oral mucosa: primarily tightness and dryness as normal responses to psychological stress.

Hence, in an embodiment, the exogenous stress is selected from chemical aggressions, mechanical aggressions, environmental aggressions, aggressions triggered by foods and/or beverages and social relationships and/or psychological issues.

In a preferred embodiment, the exogenous stress is selected from chemical aggressions or mechanical aggressions, and more preferably from chemical aggressions.

In a preferred embodiment, the chemical aggression is induced by cosmetic ingredients and preferably induced by surfactants.

For instance, chemical aggressions can be exemplified by the temporary sensations experienced by healthy oral mucosa when exposed to surfactants such as Sodium Lauryl Sulfate (SLS), commonly used in oral care products like toothpaste and mouthwash for their foaming properties. When a healthy individual uses such products, the following non-pathological physical sensations may occur:

Initially, the contact between the surfactant and the healthy oral mucosa may trigger a tingling sensation, characterized by a mild prickling feeling on the surface of the mucosa. This is typically followed by a temporary burning sensation, experienced as a mild warmth or slight stinging, particularly on the tongue and inner cheeks. These sensations are completely reversible and represent a normal response of healthy oral mucosa to surfactants.

As the product continues to be used, some individuals may experience a temporary sensation of tightness in their oral mucosa, particularly noticeable on the inner cheeks and palate. This sensation is due to the temporary interaction between the surfactant and the surface of the healthy mucosa.

Consequently, there is a clear need for soothing agents that can provide immediate comfort by alleviating these non-pathological physical sensations. Such soothing agents can help maintain the comfort of healthy individuals during their daily oral care routine. It is important to emphasize that these soothing agents are not intended to prevent or treat any pathological condition, but rather to enhance the cosmetic experience by minimizing temporary sensations that may occur during normal use of oral care products.

### Endogenous stress

Endogenous stress refers to internal factors within the body that can cause temporary non-pathological physical sensations to healthy skin or oral mucosa without leading to any pathological conditions. These internal factors can include hormonal secretions, age, anxiety, sweating and psychological mood swings.

Hormonal secretions are fluctuations in hormone levels, such as those occurring during normal life stages like menstruation, pregnancy, or menopause, that can lead to temporary sensations in healthy tissues:
- In healthy skin, these hormonal variations typically manifest as temporary sensations of tightness or mild itching, particularly noticeable during hormonal cycles,
- In healthy oral mucosa, these same hormonal fluctuations can lead to temporary sensations of dryness and occasional sensitivity, representing normal tissue responses to hormonal changes.

The internal factor "age" is the natural aging process that affects both healthy skin and oral mucosa, leading to temporary sensations that reflect normal tissue maturation:
- In healthy skin, this manifests as temporary sensations of tightness and occasional mild dryness, particularly noticeable during daily activities,
- In healthy oral mucosa, aging can lead to temporary sensations of dryness and occasional changes in comfort level, all within the normal bounds of tissue response to natural aging.

Anxiety is a psychological stress that can manifest physically in both healthy skin and oral mucosa:
- In healthy skin, anxiety typically triggers temporary sensations of warmth and occasional tingling, particularly during stressful situations,
- In healthy oral mucosa, anxiety can lead to temporary sensations of dryness and occasional tightness, representing normal physiological responses to emotional stress.
- Sweating is an increased perspiration, whether due to physical activity, environmental conditions, or emotional responses, that can affect both healthy skin and oral mucosa: In healthy skin, perspiration typically leads to temporary tingling or itching sensations, particularly in areas where sweat accumulates,
- In healthy oral mucosa, increased salivation can result in temporary changes in oral comfort.

Psychological mood swings are emotional fluctuations that can impact both healthy skin and oral mucosa through normal physiological responses:
- In healthy skin, mood changes typically manifest as temporary sensations of warmth and occasional mild itching,
- In healthy oral mucosa, these emotional variations can lead to temporary changes in comfort level and occasional sensations of dryness.

In an embodiment, the soothing agent creates or improves the physical and/or mental, particularly psychological, sensation of well-being.

This means that the agent not only alleviates temporary non-pathological physical sensations such as burning sensations, warmth, tightness, tingling, numbness, and/or itching but also enhances the overall sense of comfort and relaxation. The soothing agent achieves this by providing a pleasant sensory experience that contributes to a feeling of well-being, without any therapeutic or preventive medical purpose.

### Method for non-therapeutical soothing care

The present invention also relates to a method for a non-therapeutical soothing care for the skin and/or oral mucosa comprising applying a 1,4:3,6-dianhydrohexitol, preferably isosorbide, as soothing agent on the skin and/or oral mucosa.

The present invention also relates to a method for a non-therapeutical soothing care for the skin and/or oral mucosa comprising applying an effective amount of 1,4:3,6-dianhydrohexitol as soothing agent on the skin and/or oral mucosa of a subject in need thereof.

"An effective amount" refers to a quantity of a substance, in this case, a 1,4:3,6-dianhydrohexitol, preferably isosorbide, that is sufficient to achieve the desired soothing effect on the skin and/or oral mucosa without causing any adverse effects. This amount is determined based on the specific formulation and application method, ensuring that the soothing agent provides the intended comfort and relief from temporary non-pathological physical sensations such as burning sensations, warmth, tightness, tingling, numbness, and/or itching.

"Subject in need thereof" refers to an individual who is experiencing temporary non-pathological physical sensations, such as burning sensations, warmth, tightness, tingling, numbness, and/or itching, in their skin and/or oral mucosa. This individual is healthy and free from any pathological conditions or diseases but requires relief from these temporary discomforts caused by everyday environmental or lifestyle factors

In an embodiment the skin and the oral mucosa are healthy skin and healthy oral mucosa.

In an embodiment, the skin and/or the oral mucosa present one or more non-pathological physical sensations chosen from burning sensations, warmth, tightness, tingling, numbness, and/or itching.

In an embodiment, the non-pathological physical sensations are induced by endogenous and/or exogenous stress.

In an embodiment, the 1,4:3,6-dianhydrohexitol, preferably isosorbide, is applied on the skin or on the oral mucosa before, during or after the occurrence of endogenous and/or exogenous stress.

In an embodiment, the 1,4:3,6-dianhydrohexitol, preferably isosorbide, is comprised in a cosmetic composition.

All the previous disclosed embodiments are encompassed in this aspect.

### Cosmetic compositions

The present invention also relates to a non-therapeutic use of a 1,4:3,6-dianhydrohexitol, preferably isosorbide, as a soothing agent, wherein the 1,4:3,6-dianhydrohexitol is comprised in a cosmetic composition.

A "cosmetic composition" refers to a formulation intended for application to the skin or oral mucosa for the purpose of reducing or eliminating at least one non pathological physical sensation chosen from burning sensations, warmth, tightness, tingling, numbness, and/or itching of healthy skin or healthy oral mucosa. Such compositions are designed for non-therapeutic use and do not aim to treat or prevent any diseases or pathological conditions. They may include various ingredients such as emollients, moisturizers, surfactants, fragrances, and active agents like a 1,4:3,6-dianhydrohexitol, preferably isosorbide, which provide soothing effects and alleviate temporary non pathological physical sensations.

For healthy skin application, the soothing agent can be applied as leave-on applications. Hence, cosmetic compositions can be chosen among:
- Day creams, lotions, or balms for daily comfort,
- Night creams or masks for extended soothing during sleep,
- Protective films forming a temporary barrier,
- Patches for targeted areas requiring extended contact,
- Sprays leaving a protective layer,
- Roll-on products for precise application,
- Stick formulations for easy application.

For healthy skin application, the soothing agent can be applied as rinse-off applications. Hence, cosmetic compositions can be chosen among:
- Cleansing products with soothing properties,
- Wash-off masks,
- Shower gels or body washes,
- Bath additives for full-body comfort,
- Scrubs with soothing after-effects.

For healthy oral mucosa application, the soothing agent can be applied as leave-on applications. Hence, cosmetic compositions can be chosen among:
- Oral gels that adhere to mucosa,
- Night-time oral coating products,
- Oral films forming a temporary protective barrier,
- Oral patches for extended contact,
- Oral sprays leaving a protective layer,
- Lozenges for extended release,
- Chewing gum formulations.

For healthy oral mucosa application, the soothing agent can be applied as rinse-off applications. Hence, cosmetic compositions can be chosen among:
- Mouthwashes with various contact times (30 seconds to 2 minutes),
- Gargling solutions,
- Oral rinses of varying concentrations,
- Toothpaste formulations.

In an embodiment, the 1,4:3,6-dianhydrohexitol, preferably isosorbide, is applied through cosmetic compositions including leave-on applications and rinse-off applications:

### Cosmetic compositions for healthy skin:

Cosmetic compositions comprising a 1,4:3,6-dianhydrohexitol, preferably isosorbide, for leave-on applications include day cream, night cream, lotion, balm, protective film, patch, spray, roll-on, stick, serum, butter, oil, gel, ointment, moisturizing mask, overnight mask, comfort pad, protective barrier cream, cooling patch.

Cosmetic compositions comprising a 1,4:3,6-dianhydrohexitol, preferably isosorbide, for rinse-off applications include cleansing cream, wash-off mask, shower gel, body wash, bath additive, scrub, cleansing foam, rinse-off lotion, washing cream, comfort cleanser, foaming wash, bath oil, cleansing balm, washing gel, comfort bath powder.

### Cosmetic compositions for healthy mucosa:

Cosmetic compositions comprising a 1,4:3,6-dianhydrohexitol, preferably isosorbide, for leave-on applications include oral gel, oral film, oral patch, oral spray, lozenge, chewing gum, oral coating product, mucoadhesive film, sustained-release oral comfort strip, long-lasting oral barrier, oral comfort pad, protective oral layer, slow-dissolving oral tablet, oral comfort strip, night-time oral coating.

Cosmetic compositions comprising a 1,4:3,6-dianhydrohexitol, preferably isosorbide, for rinse-off applications include mouthwash, gargle solution, oral rinse, toothpaste, mouth refresher, oral comfort wash, cleansing oral solution, oral comfort rinse, foaming oral cleanser, oral refreshing wash, dental comfort rinse, oral cleansing foam, comfort oral wash, oral refreshing solution, oral comfort mousse.

In an embodiment, a 1,4:3,6-dianhydrohexitol, preferably isosorbide, is applied as a single application for immediate comfort of the healthy skin and/or healthy oral mucosa. Hence, in an embodiment, a 1,4:3,6-dianhydrohexitol, preferably isosorbide, is administered once at the time of experiencing the non-pathological physical sensation.

In an embodiment, a 1,4:3,6-dianhydrohexitol, preferably isosorbide, is applied through repeated applications throughout the day on the healthy skin and/or healthy oral mucosa. Hence, in an embodiment, a 1,4:3,6-dianhydrohexitol, preferably isosorbide, is administered at regular intervals to maintain comfort of the healthy skin and/or oral mucosa.

In an embodiment, a 1,4:3,6-dianhydrohexitol, preferably isosorbide, is applied as an extended overnight application healthy skin and/or healthy oral mucosa. Hence, in an embodiment isosorbide is administered before sleep and remains in contact with the healthy skin and/or oral mucosa throughout the night.

In an embodiment, the compound is applied as a pre-exposure protective application of healthy skin and/or healthy oral mucosa. Hence, in an embodiment, a 1,4:3,6-dianhydrohexitol, preferably isosorbide, is administered before anticipated exposure to factors potentially causing non-pathological physical sensations.

In an embodiment, a 1,4:3,6-dianhydrohexitol, preferably isosorbide, is applied as a post-exposure soothing application on healthy skin and/or healthy oral mucosa. Hence, in an embodiment, the soothing agent is administered immediately after exposure to factors causing non-pathological physical sensations.

In an embodiment, the compound is applied as an intermittent intensive application on healthy skin and/or healthy oral mucosa. Hence, in an embodiment, the soothing agent is administered for defined periods (for example, 15-minute application several times per day) to provide enhanced comfort during specific times.

In an embodiment of the non-therapeutic soothing care method, the exogenous stress is a chemical aggression, particularly of the oral mucosa.

In an embodiment related to chemical aggressions from oral care products, the compound is applied as a protective oral film before tooth brushing, followed by a soothing mouth rinse (30 seconds to 1 minute) immediately after brushing, and completed with an adherent oral gel providing extended comfort for 1 to 2 hours.

In an embodiment, the composition comprises from 0.1 to 50% by weight, preferably from 0.1 to 30% by weight, preferably from 0.2 to 15% by weight, preferably from 0.5 to 7% by weight, based on the total weight of the composition, of a 1,4:3,6-dianhydrohexitol, preferably isosorbide.

In an embodiment, the 1 ,4:3,6-dianhydrohexitol, preferably isosorbide, is added to a cosmetic composition comprising an ingredient responsible for a chemical aggression of the skin and/or the oral mucosa, and thus exert its soothing effect simultaneously to the chemical aggression, thereby reducing or suppressing at least one of the non-pathological sensations chosen from burning sensation, warmth, tightness, tingling, numbness and/or itching, that said chemical aggression caused to the skin and/or to the oral mucosa.

### Embodiments

Embodiment 1: a non-therapeutic use of a 1,4:3,6-dianhydrohexitol as a soothing agent.

Embodiment 2: the non-therapeutic use according to embodiment 1, wherein the soothing agent is for skin and/or oral mucosa.

Embodiment 3: The non-therapeutic use according to embodiment 2, wherein the soothing agent is for oral mucosa.

Embodiment 4: The non-therapeutic use according to any of embodiments 1 to 3, wherein the soothing agent reduces or eliminates at least one non-pathological physical sensation chosen from burning sensations, warmth, tightness, tingling, numbness, and itching.

Embodiment 5: The non-therapeutic use according to embodiment 4, wherein the non-pathological physical sensation is induced by endogenous and/or exogenous stress.

Embodiment 6: The non-therapeutic use according to embodiment 5, wherein the exogenous stress is selected from chemical aggressions, mechanical aggressions, environmental aggressions, aggressions triggered by foods and/or beverages, social relationships and/or psychological issues.

Embodiment 7: The non-therapeutic use according to embodiment 6, wherein the exogenous stress is selected from chemical aggressions or mechanical aggressions, and preferably from chemical aggressions.

Embodiment 8: The non-therapeutic use according to embodiment 7, wherein the chemical aggression is induced by cosmetic ingredients and preferably induced by surfactants.

Embodiment 9: The non-therapeutic use according to embodiment 5, wherein the endogenous stress is chosen from hormonal secretions, age, anxiety, sweating, and/or psychological mood swings.

Embodiment 10: The non-therapeutic use according to any of embodiments 1 to 3, wherein the soothing agent creates or improves the physical and/or mental sensation of well-being.

Embodiment 11: A method for a non-therapeutical soothing care for the skin and/or oral mucosa comprising applying a 1,4:3,6-dianhydrohexitol as a soothing agent on the skin and/or oral mucosa.

Embodiment 12: The method for a non-therapeutical soothing care according to embodiment 11, wherein the 1,4:3,6-dianhydrohexitol is applied on skin and/or oral mucosa presenting one or more non-pathological physical sensation chosen from burning sensations, warmth, tightness, tingling, numbness, and itching.

Embodiment 13: The method for a non-therapeutic soothing care according to embodiment 12, wherein the physical sensation is induced by endogenous and/or exogenous stress.

Embodiment 14: The method for non-therapeutic soothing care according to embodiment 13, wherein the 1,4:3,6-dianhydrohexitol is applied on skin and/or oral mucosa, preferably on oral mucosa before, during or after the occurrence of an endogenous and/or exogenous stress.

Embodiment 15: A non-therapeutic use according to any of embodiments 1 to 10, or a method for non-therapeutical soothing care according to any of claims 11 to 14, wherein the 1,4:3,6-dianhydrohexitol is isosorbide

## Claims

1. A non-therapeutic use of a 1,4:3,6-dianhydrohexitol as a soothing agent.

2. The non-therapeutic use according to claim 1, wherein the soothing agent is for skin and/or oral mucosa.

3. The non-therapeutic use according to claim 2, wherein the soothing agent is for oral mucosa.

4. The non-therapeutic use according to any of claims 1 to 3, wherein the soothing agent reduces or eliminates at least one non-pathological physical sensation chosen from burning sensations, warmth, tightness, tingling, numbness, and itching.

5. The non-therapeutic use according to claim 4, wherein the non-pathological physical sensation is induced by endogenous and/or exogenous stress.

6. The non-therapeutic use according to claim 5, wherein the exogenous stress is selected from chemical aggressions, mechanical aggressions, environmental aggressions, aggressions triggered by foods and/or beverages, social relationships and/or psychological issues.

7. The non-therapeutic use according to claim 6, wherein the exogenous stress is selected from chemical aggressions or mechanical aggressions, and preferably from chemical aggressions.

8. The non-therapeutic use according to claim 7, wherein the chemical aggression is induced by cosmetic ingredients and preferably induced by surfactants.

9. The non-therapeutic use according to claim 5, wherein the endogenous stress is chosen from hormonal secretions, age, anxiety, sweating, and/or psychological mood swings.

10. The non-therapeutic use according to any of claims 1 to 3, wherein the soothing agent creates or improves the physical and/or mental sensation of well-being.

11. A method for a non-therapeutical soothing care for the skin and/or oral mucosa comprising applying a 1,4:3,6-dianhydrohexitol as a soothing agent on the skin and/or oral mucosa.

12. The method for a non-therapeutical soothing care according to claim 11, wherein the 1,4:3,6-dianhydrohexitol is applied on skin and/or oral mucosa presenting one or more non-pathological physical sensation chosen from burning sensations, warmth, tightness, tingling, numbness, and itching.

13. The method for a non-therapeutic soothing care according to claim 12, wherein the physical sensation is induced by endogenous and/or exogenous stress.

14. The method for non-therapeutic soothing care according to claim 13, wherein the 1,4:3,6-dianhydrohexitol is applied on skin and/or oral mucosa, preferably on oral mucosa before, during or after the occurrence of an endogenous and/or exogenous stress,

15. A non-therapeutic use according to any of claims 1 to 10, or a method for non-therapeutical soothing care according to any of claims 11 to 14, wherein the 1,4:3,6-dianhydrohexitol is isosorbide.
